# EUROPEAN PATENT APPLICATION

(11) **EP 3 488 776 A1**
(43) Date of publication of application: **29.05.2019**
(21) Application number: 17203216.1
(22) Date of filing: 23.11.2017
(51) Int. Cl.: A61B 5/024, A61B 5/00

(54) **WEARABLE DEVICE USING PPG SENSOR FOR OPTICAL COMMUNICATION**

(71) Applicant: Koninklijke Philips N.V., 5656 AE Eindhoven (NL)
(72) Inventor: GELISSEN, Jozef Hubertus, 5656 AE Eindhoven (NL); HAAKMA, Reinder, 5656 AE Eindhoven (NL); GEENEN, Koen, 5656 AE Eindhoven (NL); ROMMERS, Adrianus Petrus Johanna Maria, 5656 AE Eindhoven (NL)
(74) Representative: de Haan, Poul Erik

(57) **Abstract**

The present invention relates to the field of medical technology as well as fitness and activity trackers. A wearable device adapted to acquire physiological data of a subject (100) is presented, the wearable device comprising a photo plethysmography, PPG, sensor (20) comprising at least one light source (21) and at least one photodetector (24); wherein the wearable device is further adapted to transmit data optically via said at least one light source (21) of the PPG sensor (20). It is thus proposed that the PPG sensor is not only used for acquisition of a physiological signal but also used for optical communication, in particular for offloading acquired physiological data from the wearable device. The present invention further relates to a corresponding docking station, system and method.

## Description

### FIELD OF THE INVENTION

The present invention relates to the fields of medical technology as well as fitness and activity trackers and, in particular, to a wearable device adapted to be worn by a user and adapted to acquire physiological data of a subject. The wearable device comprises a PPG (photo plethysmography) sensor comprising at least one light source and at least one photodetector. The present invention further relates to a corresponding docking station, system, method and computer program.

### BACKGROUND OF THE INVENTION

Vital signs of a person, for example the heart rate (HR), the respiration rate (RR) or the arterial blood oxygen saturation (SpO2), serve as indicators of the current health or fitness state of a person and as powerful predictors for serious medical events. For this reason, vital signs are extensively monitored in inpatient and outpatient care settings, at home or in further health, leisure and fitness settings.

Recently, wearable devices such as activity trackers, sports watches, and health watches have been developed that measure health parameters or physiological signals such as the heart rate using photo plethysmography (PPG). An exemplary wearable device that is commercially available is the Philips health watch, e.g. model DL8790/00, of the applicant.

Physiological data indicative of a physiological signal that has been acquired by wearable devices is typically transferred e.g. to a smart phone or computer for presentation to the user and/or further analysis. A convenient way of transferring data is using RF (radio frequency) wireless communication technology such as WiFi or Bluetooth. In order to reduce the power consumption, Bluetooth Low Energy has been widely adopted, however, at the cost of a lower data rate.

### SUMMARY OF THE INVENTION

It is an object of the present disclosure to provide an improved wearable device. In particular, it would be advantageous to provide a wearable device that improves offloading acquired physiological data from the wearable device.

In a first aspect of the present disclosure a wearable device adapted to acquire physiological data of a subject is presented that comprises a PPG sensor comprising at least one light source and at least one photodetector; wherein the wearable device is further adapted to transmit data optically via said at least one light source of the PPG sensor or to receive data optically via said at least one photodetector of the PPG sensor. In other words, the wearable device is adapted to perform optical communication using at least one of the at least one light source and the at least one photodetector of the PPG sensor.

In a further aspect of the present disclosure a docking station for the aforementioned wearable device is presented, wherein the docking station is adapted to perform optical communication with the wearable device via at least one of the at least one light source and the at least one photodetector of the PPG sensor of the wearable device.

In a further aspect of the present disclosure a system is presented comprising
a wearable device adapted to acquire physiological data of a subject, the device comprising a PPG sensor comprising at least one light source and at least one photodetector; wherein the wearable device is further adapted to transmit data optically via said at least one light source of the PPG sensor or to receive data optically via said at least one photodetector of the PPG sensor; and
a terminal adapted to receive physiological data transmitted optically via the at least one light source of the PPG sensor of the wearable device or to transmit data optically to the at least one photodetector of the PPG sensor, respectively. In other words, the wearable device and the terminal are adapted to perform optical communication via at least one of the at least one light source and the at least one photodetector of the PPG sensor of the wearable device.

In another aspect of the present disclosure, an alignment adapter for use in the aforementioned system is disclosed, wherein the alignment adapter comprises a seat for receiving the wearable device and wherein the alignment adapter is adapted to provide an optical transmission path between at least one of (i) a light source of a PPG sensor of the wearable device and a photodetector of the terminal and (ii) a photodetector of the PPG sensor of the wearable device and a light source of the terminal. The alignment adapter may optionally be part of the system.

In yet another aspect of the present disclosure, a method for acquiring and transmitting physiological data of a subject is presented, the method comprising the steps of: acquiring physiological data of a subject using a PPG sensor comprising at least one light source and at least one photodetector; and performing optical communication using the at least one light source of the PPG sensor. The optical communication may comprise transmitting at least part of the physiological data of the subject. A wearable device may be used for acquisition of the physiological data and the physiological data may be stored in a memory of the wearable device.

In yet further aspects of the present invention, there are provided a corresponding computer program comprising program code means for causing a processor to carry out the step of controlling a light source of a PPG sensor to optically transfer data using the same light source used for measurement of a physiological parameter of a subject; as well as a non-transitory computer-readable recording medium that stores therein a computer program product, which, when executed by a processor, causes the processor to carry out the aforementioned step.

Preferred embodiments of the invention are defined in the dependent claims. It shall be understood that the claimed method, docking station, system, computer program and medium may have similar and/or identical preferred embodiments as the claimed wearable device, in particular as defined in the dependent claims and as disclosed herein.

The present invention is based on the idea to use an already existing PPG (photo plethysmography) sensor of the wearable device for optical data transmission, thereby providing a synergistic effect. The same light source and/or photodetector that are already used for measurement of a physiological signal to acquire the physiological data of the subject are also used for optical communication e.g. for offloading the physiological data from the wearable device.

The proposed solution may provide one or more of the following advantages:
(i) weight reduction, since no additional electronics for wireless communication are required;
(ii) cost reduction and (iii) size reduction for the same reason; (iv) increased data rate, in particular overcoming data rate limitations of Bluetooth Low Energy; (v) improved physical link security regarding data transmission since visual contact is required for optical data transmission, i.e., no interception of RF radiation; (vi) improved esthetics compared to contact-based electrical data interface since no additional electrical connectors are required, in particular if the wearable device is adapted for inductive charging; (vii) reduced RF interference potential as well as improved acceptance of people concerned with RF radiation. Moreover, the certification process regarding RF compliance may be facilitated.

It should further be noted that the proposed solution may directly benefit from the ever increasing speeds of the electronics of wearable devices and optical receivers since it is not tied to the limitations of the Bluetooth Low Energy standard. In order to improve compatibility with further devices, the wearable device may be adapted to perform optical communication via the light source of the PPG sensor in compliance with Li-Fi technology or related standards.

Generally, wearable devices are able to gather massive amounts of data. In prior art wearable devices the acquired data may have to be reduced or compressed before offloading. For example, only average values or extracted values such as the heart rate are transmitted. Furthermore, a sampling rate may be reduced. For example, prior art physiological data may include motion signals at a limited sampling rate of e.g. no more than 1 Hz. Even in this case, if synchronization of the wearable device has not been performed for e.g. several days, offloading the data from the wearable device using Bluetooth Low Energy may take several minutes. However, it would be desirable to increase the throughput. In particular, it would be desirable to transfer not only average values but additional data e.g. raw data such as the shape of the waveform of a blood absorption signal measured by the PPG sensor to enable more sophisticated data analysis. Hence, the size of the data to be transferred may increase even further.

As used herein, physiological data of the subject can be indicative of a health parameter or vital sign of the subject. For example, physiological data can be indicative of at least one of a heart rate (HR), respiration rate (RR), and/or blood oxygen saturation (SpO2). The acquired physiological data may correspond to a measured physiological signal. The physiological signal may be a waveform based on which a health parameter or vital sign may be derived.

Performing optical communication using the at least one light source of the PPG sensor may comprise transmitting at least part of the physiological data of the subject. The physiological data may refer to physiological data acquired with said PPG sensor. The acquired physiological data may be stored in a memory of the wearable device.

As used herein, a docking station may refer to a device in or on which the wearable device may be placed for offloading physiological data of the subject that has been acquired with the wearable device. Optionally, the docking station may be used for charging, providing access to a power supply and/or to peripheral devices or auxiliary features.

As used herein, a terminal or terminal device refers to a device that is adapted to receive physiological data transmitted optically via the at least one light source of the PPG sensor of the wearable device. The terminal may be a docking station. However, the terminal may more generally also refer to other devices, for instance a smart phone comprising a photodetector, wherein the smartphone is adapted for uni or bidirectional optical communication with the wearable device. An already existing photodetector of the terminal device may be used, for example an ambient light detector, at least one element of a camera sensor, a built-in PPG detector. A computer program or a (smartphone) app may be provided to extract the data transmitted from the wearable device in the detected optical signal.

Optionally, the wearable device may be further adapted to receive data optically via said at least one photodetector of the PPG sensor. An advantage of this embodiment is that, the wearable device can perform bidirectional optical communication.

In an embodiment, the wearable device may comprise a modulator for modulating a driving current or voltage of the at least one light source of the PPG sensor. An advantage of this embodiment is that such a direct modulation of the light source may be implemented at low cost with low complexity. The light emitted by the light source is thus modulated based on data to be transmitted. The modulator may comprise electronic switches such as field effect transistors (FETs) adapted to switch the light source of the PPG sensor on and off. Data transmission formats known from optical communication technology such as on-off-keying can be used. The wearable device may comprise an analog frontend for PPG measurement and communication electronics that can be selectively coupled to the at least one light source. Correspondingly, the analog frontend for PPG measurement and the communication electronics may be selectively coupled to the at least one photodetector.

Optionally, the at least one light source of the PPG sensor may be an LED (light emitting diode) or a laser diode, in particular a low capacitance LED or low capacitance laser diode. Optoelectronics companies often provide, in addition to standard devices, so-called low-capacitance light sources that are adapted to be modulated at high switching frequencies since they are designed to provide a low capacitance. An advantage of this embodiment is that higher data rates can be achieved. Furthermore, the power consumption can be reduced since less current is required for charging and discharging the light source capacitance.

Optionally, the wearable device may be adapted to encode (and/or decode) data using Manchester coding. An advantage of this embodiment is that the Manchester code is robust to DC shifts. Such DC shifts may occur due to stray light.

Optionally, the wearable device may further be adapted to switch between a measurement mode for acquisition of physiological data of the subject using the PPG sensor and a data transmission mode for transmitting data using the PPG sensor. The wearable device may comprise a control unit adapted to switch between the measurement mode and the data transmission mode. Hence, dedicated operational modes of the PPG sensor may be provided. The PPG sensor may preferably not acquire physiological data during data transmission mode.

Optionally, the wearable device may be adapted to switch between said measurement mode and said data transmission mode based on at least one of a user input and charging of the wearable device. For example, a user input via a user interface may set the wearable device in a certain mode. In addition or in the alternative, the wearable device may be set to data transmission mode when it is detected that the wearable device is charged. For example, the wearable device may switch to optical data transmission mode when placed on a docking station.

Optionally, the wearable device may be adapted to switch between said measurement mode and said data transmission mode based on an optical trigger signal received via the at least one photodetector of the PPG sensor. An advantage of this embodiment may be an efficient implementation. In particular, a PPG sensor that is connected to communication circuitry may directly be used for initiating the communication. The optical trigger signal may be an optical handshake signal. The handshake signal may include additional information, e.g. regarding data rates or transmission protocols to be used e.g. for offloading physiological data from the wearable device.

Optionally, the wearable device may be adapted to switch between said measurement mode and said data transmission mode based on a physiological sensor modality of the wearable device. An advantage is that an (already existing) physiological sensor modality of the wearable device may be used for switching so that no additional element is required. For example, a bio-impedance, temperature, ECG signal, PPG signal, etc. can be evaluated. If it is determined that a measurement value of a physiological sensor modality crosses a threshold value or is within a predetermined range or if a morphology of a corresponding physiological signal fulfills a predetermined condition, the wearable device may switch between said measurement mode and said data transmission mode. For example, it may be determined that the physiological signal corresponds to an expected template. Optionally, a docking station or terminal may be adapted to provide a predetermined stimulus to the physiological sensor modality of the wearable device to trigger the data transmission mode. For example, the docking station or terminal may provide a stimulus at a predetermined value, above or below a predetermined threshold, according to a predetermined template such as a sinus signal at a predetermined frequency.

Optionally, the PPG sensor may comprise an infrared light source and the wearable device may be adapted to transmit data optically via said infrared light source. An advantage of this embodiment is that invisible radiation may be used for transmitting data so as not to disturb the user. This may be particularly advantageous if the wearable device is placed on a nightstand next to a bed of the user.

Optionally, the PPG sensor may comprises a first light source operating at a first wavelength and a second light source operating at a second wavelength; wherein said first wavelength is different from said second wavelength; and wherein the device may be adapted for dual-channel data transmission at said first and said second wavelength. An advantage of this embodiment can be a further increased data rate. For example, the transmission rate may be doubled without increasing the switching frequency for modulating each individual light source. The different wavelengths may be different wavelengths used for pulse oximetry, such as green and infrared light. Hence, already existing light sources of a PPG sensor may advantageously be reused for communication. Correspondingly, a receiver or docking station may be provided with first and second photodetectors adapted to selectively receive light from the first and second light sources. For example, the first and second photodetectors may be equipped with spectral filters to separate the data streams. In addition or in the alternative, the PPG sensor may comprise a first and a second light source, wherein the wearable device may be further adapted for dual-channel data transmission via optically isolated transmission paths using said first and said second light source. In this case it is not necessary to use different wavelengths.

The aforementioned docking station or terminal device may comprise a photodetector or receiver for receiving data transmitted optically via at the least one light source of the PPG sensor of the wearable device. The photodetector is arranged to be opposite to the light source of the wearable device when the wearable device is placed on the docking station or terminal device.

Optionally, the aforementioned docking station or terminal device may comprise a light source for optical communication with the wearable device. An advantage of this embodiment is that bidirectional optical communication can be performed. In addition or in the alternative, the light source may be used to send a trigger signal or a handshake signal to the wearable device to initiate data transfer.

Optionally, the docking station or terminal device may comprise a light shield adapted to reduce stray light in a path between the at least one light source of the wearable device and a photodetector of the docking station or terminal device. Stray light as used herein may refer to ambient light such as for example sunlight or room illumination. In addition or in the alternative, the light shield may also reduce undesired stray light from one or more additional optical communication paths between the wearable device and the docking station.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a wearable device comprising a PPG sensor;
Fig. 2 shows a schematic diagram of a wearable device in measurement mode attached to a wrist of a subject;
Fig. 3A and 3B show a schematic diagrams of a wearable device in data transmission mode placed on a docking station;
Fig. 4 shows a block diagram of an embodiment of a wearable device;
Fig. 5 shows a schematic diagram of a wearable device placed on a docking station or terminal;
Fig. 6 shows a schematic diagram of a wearable device placed on a terminal with the use of an alignment adapter;
Fig. 7 shows a schematic diagram of the alignment adapter of Fig. 6; and
Fig. 8 shows a flow chart of a method for acquiring and transmitting physiological data of a subject.

### DETAILED DESCRIPTION OF THE INVENTION

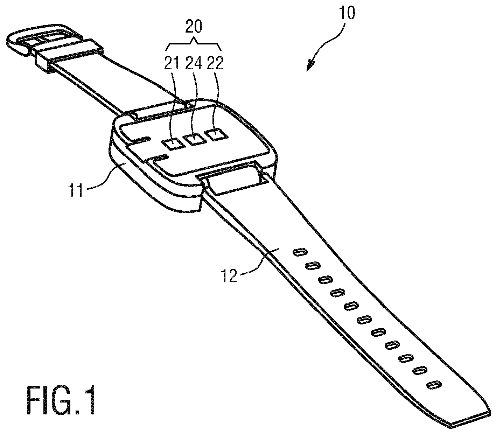

Fig. 1 schematically shows a first embodiment of a wearable device according to an embodiment of the present invention. The wearable device is therein denoted in its entirety by reference numeral 10. In the given embodiment, the wearable device is implemented in form of a wrist-worn device comprising a housing 11 and fixation means in form of a wrist-band 12. However, the wearable device may also be implemented in other forms, such as for example a patch that can be applied to the subject.

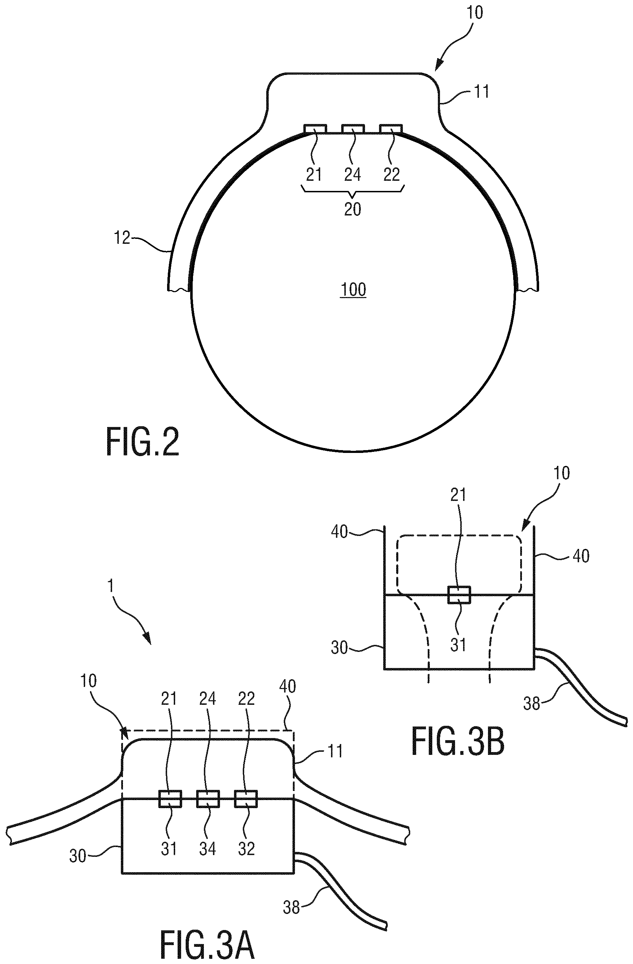

Fig. 2 shows a schematic diagram of the wearable device 10 attached to a wrist of a subject 100. In the given example, the wearable device may also be referred to as a fitness tracker or health watch. The wearable device 10 is adapted to acquire physiological data of the subject 100. The wearable device comprises a PPG (photo plethysmography) sensor 20 that is preferably arranged at a lower side of the housing 11 and adapted contact a skin of the subject 100. The PPG sensor comprises at least one light source (21) and at least one photodetector (24).

Plethysmography generally refers to the measurement of volume changes of an organ or a body part and in particular to the detection of volume changes due to a cardiovascular pulse wave travelling through the body of a subject with every heartbeat. Photo plethysmography (PPG) is an optical measurement technique that evaluates a time-varying change of light reflectance or transmission of an area or volume of interest. PPG is based in the principle that blood absorbs light more than surrounding tissue, so that variations in blood volume with every heart beat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared or green and infrared), the blood oxygen saturation (SpO2) can be determined.

In the given example, the wearable device comprises a first light source 21 and a second light source 22. The first light source 21 may be operating at first wavelength and the second light source 22 may be operating at a second wavelength; wherein said first wavelength is different from said second wavelength. Preferably, the first and second wavelengths are adapted to enable an SpO2 measurement, e.g. red and infrared or green and infrared light sources.

As illustrated in Fig. 2, the first light source 21 emits light at a first wavelength towards a skin of the subject 100 which is partially absorbed and partially scattered back to the photodetector 24. Correspondingly, the second light source 22 emits light at a second wavelength towards the skin of the subject 100 which is also partially absorbed a partially scattered back to the photodetector. The detected light intensity at the first and/or second wavelength provides a physiological signal trace that is indicative of the underlying physiological phenomenon and can be stored as physiological data of the subject in a memory or storage of the wearable device 10. Insofar, the operation corresponds to a known operation of a PPG sensor of a wearable device. This mode of operation can also be referred to as a so-called measurement mode for acquisition of physiological data of the subject 100 using the PPG sensor 20.

According to an aspect of the present disclosure, however, the wearable device is further adapted to transmit data optically via said at least one light source (21) of the PPG sensor (20) and/or to receive data optically via said at least one photodetector (24) of the PPG sensor (20). For example, the wearable device may be adapted to switch between a measurement mode for acquisition of physiological data and a data transmission mode for transmitting data using the PPG sensor 20. In other words, the same PPG sensor that is used for acquisition of the physiological data may also be used for receiving data or offloading data from the wearable device by optical communication.

Fig. 3A and 3B show a schematic diagrams of a wearable device 10 in data transmission mode placed on a terminal in form of a docking station 30. Fig. 3A shows a side view of the wearable device 10 placed on the docking station 30. Fig. 3B shows a front view of the wearable device 10 placed on the docking station 30. The docking station 30 is adapted to perform optical communication with the wearable device 10 via at least one of the at least one light source 21 and the at least one photodetector 24 of the PPG sensor 20 of the wearable device 10. The docking station 30 comprises at least one photodetector 31 arranged opposite to the light source 21 of the wearable device 10 when the wearable device is placed on the docking station.

Optionally, the docking station may comprise a second photodetector 32 arranged opposite to the second light source 22 of the wearable device 10. The second photodetector 32 may also be provided if the wearable device has only one light source 21 so that light can be received from the wearable device 10, even if the wearable device is placed on the docking station rotated by 180°.

The docking station 30 may further comprise at least one light source 34 arranged opposite to the photodetector 24 of the wearable device. Thereby, the docking station 30 and the wearable device 10 may perform bidirectional optical communication.

In an embodiment, the docking station 30 may comprise a light shield 40 for shielding off stray light that might otherwise disturb the optical communication link. In the exemplary embodiment of Fig. 3A and 3B, the light shield 40 is implemented by baffles arranged at two sides of a receiving portion where the wearable device 10 is to be placed for offloading data.

The docking station 30 preferably comprises a wired or inductive charger for charging the wearable device 10 when placed on the docking station.

The docking station 30 or terminal preferably comprise a protocol converter for converting the data received via the optical communication link and for passing at least some of the received data on to a further device via a wired or wireless interface, for example via a USB cable 38. Alternatively or in addition, the docking station may be equipped with a WiFi transmitter. The system 1 may thereby overcome limitations regarding energy consumption and/or availability of wired or RF transmission capabilities of the wearable device 10. Optionally, the received data may undergo additional data analysis or processing steps in the docking station or terminal.

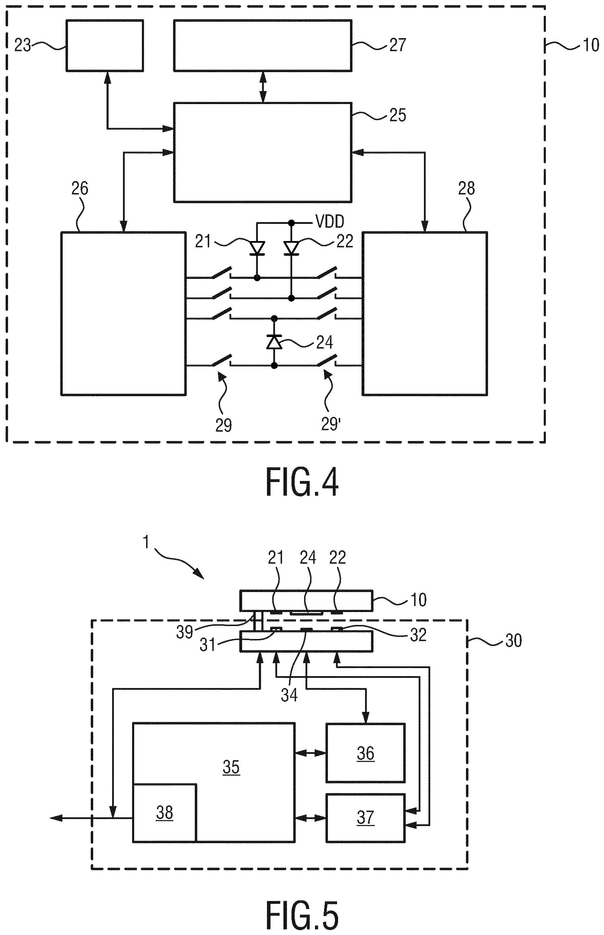

Fig. 4 shows a block diagram of an embodiment of a wearable device 10. The wearable device 10 comprises a PPG sensor comprising a first light source 21 and optionally a second light source 22 as well as a photodetector 24. The light sources may be connected to a power supply VDD. The wearable device 10 further comprises a processor 25 that is connected to a memory 27. Optionally one or more additional sensors, such as an accelerometer 23 may be provided to acquire additional physiological data of the subject. In a first, measurement mode an (analog) PPG frontend 26 may be selectively coupled to the light sources 21, 22 and the photodetector 24 for acquisition of physiological data of the subject using the PPG sensor via switches 29. In a second, data transmission or communication mode, communication electronics 28 such as modulator may be selectively coupled to at least one of the light sources 21, 22 and optionally to the photodetector 24 for performing optical communication using the PPG sensor via switch 29'. It shall be understood that the function of one or more of the elements may be implemented by means of a processer such as a microcontroller that may realize the functionality of at least some of the processor 25, the PPG frontend 26, the memory 27, the communication electronics 28 as well as the switches 29, 29'.

Data acquired by the PPG sensor and/or one or more additional sensors 23 may be stored in the memory 27. It shall be understood that the data to be transmitted via the PPG sensor is not limited to physiological data acquired by the PPG sensor but may also refer to additional or different data such as system data or data acquired by an accelerometer 23.

Fig. 5 shows a schematic diagram of a wearable device 10 placed on a docking station 30. The wearable device may for example be a wearable device as described above or a wearable device in form of a patch for application to a skin of the subject. The docking station 30 may again comprise a first photodetector 31 and optionally a second photodetector 32 and/or a light source 34 for bidirectional communication. Further, the docking station 30 may provide electrical contacts 39 such as electrical pins for charging the wearable device 10. The docking station preferably comprises a processor 35 that is connected to a modulator 36 for transmitting data to the wearable device 10 via the light source 34. The at least one photodetector 31, 32 may be connected to an optional amplifier 37 for amplifying a signal received from the photodetector. The (amplified) photodiode signal may then be received by the processor 35. For example, the processor may be implemented by a microcontroller having integrated analog-to-digital converters for receiving the output signal of the photodetectors. The processor can be adapted to demodulate the received signal to obtain the data transmitted via the light source 21 of the wearable device 10. Optionally, the docking station 30 may comprise a protocol converter 38 or communication interface that is adapted to translate the data to a different communication protocol such as USB for transmission to a computer or a WiFi transmitter for sending data e.g. to a cloud environment.

It shall be understood that a docking station 30 is an advantageous embodiment of a terminal. However, a terminal can also refer to further devices adapted to receive physiological data transmitted optically via the at least one light source (21) of the PPG sensor (20) of the wearable device (10). For example, a smartphone comprising program code for causing the smartphone to perform optical communication with the wearable device may serve as a terminal device. A light detector of the smartphone, such as brightness sensor or even at least one a pixel of a camera of the smartphone may be used as the photodetector. The light received from the at least one light source of the wearable device may then be demodulated by a processor of the smartphone to obtain the (physiological) data transmitted by the light source of the PPG sensor of the wearable device. Optionally, an already existing light source of the smartphone, advantageously a flash light of the smartphone arranged adjacent to the camera, may be used for bidirectional optical communication with the wearable device.

Optionally, a light source of the terminal may be used as a trigger signal or handshake signal to initiate data transmission by the wearable device 10.

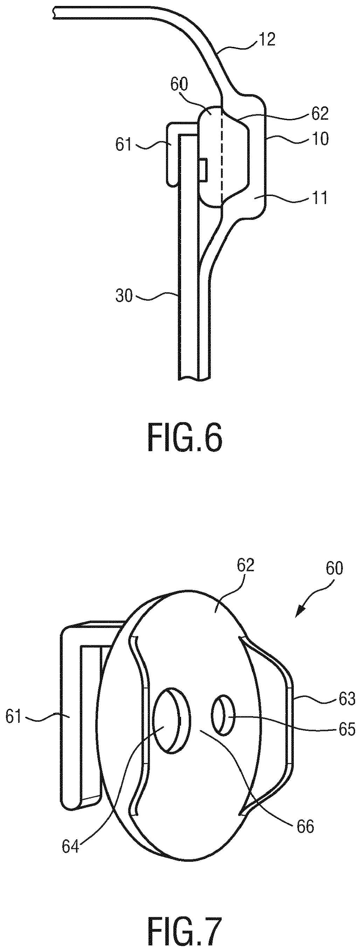

Fig. 6 shows a schematic diagram of a wearable device 10 placed on a terminal 30 with the use of an alignment adapter 60. In the give embodiment, the terminal 30 may be a smartphone. The alignment adapter 60 may comprise a fixing means such as a clip 61 for attachment to the terminal 30. The alignment adapter 60 may further comprise a seat 62 for receiving the wearable device 10. The alignment adapter is adapted to align the wearable device 10 with the terminal 30. More precisely, the alignment adapted is adapted to provide an optical transmission path between at least one of (i) a light source 21 of a PPG sensor 20 of the wearable device 10 and a photodetector 32 of the terminal 30 and (ii) a photodetector 24 of the PPG sensor 20 of the wearable device 10 and a light source 34 of the terminal 30. Referring back to Fig. 5, the alignment adapter may thus be seen as an additional element that ensures proper alignment between the wearable device 10 and the terminal 30 for optical communication between the two devices.

Fig. 7 shows a schematic diagram of the alignment adapter 60 of Fig. 6. Optionally, the alignment adapter 60 may further comprise at least one light shield 63, here in form of a baffle, to reduce or prevent stray light from reaching the optical transmission path. The alignment adapter 60 may comprise a first opening 64 to be aligned with at least one photodetector of the terminal device 30 and a light source of a PPG sensor of the wearable device 10. In addition or in the alternative, the alignment adapter 60 may comprise a second opening 65 to be aligned with a light source of the terminal device 30 and a photodetector of the PPG sensor the wearable device 10. For example, for the case of a smartphone as the terminal device 30, the first opening may be aligned with a camera of the smartphone and the second opening may be aligned with a flashlight of the smartphone. An optical shield 66 may optionally be provided therein between to separate both optical communication paths.

Optionally, an alignment adapter may comprise a light guide such as optical fiber. Hence, the alignment adapter may be adapted to provide an optical transmission path between at least one of (i) a light source (21) of a PPG sensor (20) of the wearable device (10) and a photodetector (32) of the terminal (30) and (ii) a photodetector (24) of the PPG sensor (20) of the wearable device (10) and a light source (34) of the terminal (30) by means of a light guide.

A distance between a light source 21, 22 and a photodetector 24 of the PPG sensor 20 (cf. Fig 1) may depend on the used wavelength. For example, green light (∼500nm) has a shorter wavelength than red light (∼700nm). Since red light has a higher penetration depth, a spacing between the respective light source and the photodetector may be larger. Moreover, a distance between a light source 21, 22 and a photodetector 24 may be completely different than the distance of an optical sensor 31 and light source 34 of the terminal 30. An alignment adaptor may advantageously be adapted to overcome such different positions by providing an optical transmission path via a light guide.

Optionally, an interfacing between the wearable device 10 and the docking station or terminal 30 may even be contactless or remotely. In other words, the wearable device 10 and the terminal 30 may be adapted to perform free space optical communication. For example, a focused coherent light source such as a laser may be used for remote optical communication. The light source for optical communication may again be a light source of the PPG sensor 20 and/or a photodetector of the PPG sensor 20 may receive data optically from a (focused coherent) light source of a terminal 30.

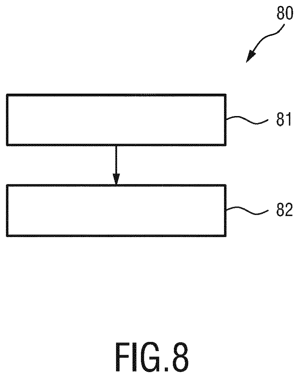

Fig. 8 shows a flowchart of a method 80 for acquiring and transmitting physiological data of a subject 100. In a first step 81, physiological data of a subject 100 is acquired using a PPG sensor 20 comprising at least one light source 21 and at least one photodetector 24. In a second step 82, optical communication is performed using the at least one light source 21 and/or the at least one photodetector 24 of the PPG sensor 20. The PPG sensor may be a PPG sensor of a wearable device as described above. The optical communication may involve transmitting or offloading physiological data via the same light source that has previously been used to acquire at least some of said physiological data.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Wearable device (10) adapted to acquire physiological data of a subject (100), the wearable device comprising a PPG sensor (20) comprising at least one light source (21) and at least one photodetector (24); wherein the wearable device is further adapted to transmit data optically via said at least one light source (21) of the PPG sensor (20) or to receive data optically via said at least one photodetector (24) of the PPG sensor (20).

2. Wearable device as claimed in claim 1, adapted to transmit data optically via said at least one light source (21) of the PPG sensor (20) and to receive data optically via said at least one photodetector (24) of the PPG sensor (20).

3. Wearable device as claimed in claim 1, comprising a modulator for modulating a driving current or voltage of the at least one light source (21) of the PPG sensor (20).

4. Device as claimed in claim 1, wherein the at least one light source (21) of the PPG sensor (20) is a low capacitance LED or low capacitance laser diode.

5. Wearable device as claimed in claim 1, wherein the device is adapted to encode data using Manchester coding.

6. Wearable device as claimed in claim 1, further adapted to switch between a measurement mode for acquisition of physiological data of the subject using the PPG sensor (20) and a data transmission mode for transmitting data using the PPG sensor (20).

7. Wearable device as claimed in claim 6, wherein the wearable device (10) is adapted to switch between said measurement mode and said data transmission mode based on at least one of a user input and charging of the wearable device.

8. Wearable device as claimed in claim 6, wherein the wearable device (10) is adapted to switch between said measurement mode and said data transmission mode based on an optical trigger signal received via the at least one photodetector (24) of the PPG sensor (20).

9. Wearable device as claimed in claim 6, wherein the wearable device (10) is adapted to switch between said measurement mode and said data transmission mode based on a physiological sensor modality of the wearable device (10).

10. Wearable device as claimed in claim 1, wherein the PPG sensor (20) comprises a first light source (21) operating at a first wavelength and a second light source (22) operating at a second wavelength; wherein said first wavelength is different from said second wavelength; and wherein the wearable device (10) is adapted for dual-channel data transmission at said first and said second wavelength.

11. Docking station (30) for a wearable device (10) as claimed in claim 1, wherein the docking station is adapted to perform optical communication with the wearable device via at least one of the at least one light source (21) and the at least one photodetector (22) of the PPG sensor (20) of the wearable device.

12. System (1) comprising:
- a wearable device (10) adapted to acquire physiological data of a subject (100), the wearable device comprising a PPG sensor (20) comprising at least one light source (21) and at least one photodetector (24); wherein the wearable device is further adapted to transmit data optically via said at least one light source (21) of the PPG sensor (20) or to receive data optically via said at least one photodetector (24) of the PPG sensor (20); and
- a terminal (30) adapted to receive physiological data transmitted optically via the at least one light source (21) of the PPG sensor (20) of the wearable device (10) or to transmit data optically to the at least one photodetector (24) of the PPG sensor (20), respectively.

13. An alignment adapter (60) for use in a system (1) according to claim 12, wherein the alignment adapter comprises a seat for receiving the wearable device (10) and wherein the alignment adapter is adapted to provide an optical transmission path between at least one of (i) a light source (21) of a PPG sensor (20) of the wearable device (10) and a photodetector (32) of the terminal (30) and (ii) a photodetector (24) of the PPG sensor (20) of the wearable device (10) and a light source (34) of the terminal (30).

14. A method (80) for acquiring and transmitting physiological data of a subject (100), the method comprising the steps of:
- acquiring physiological data of a subject using a PPG sensor (20) comprising at least one light source (21) and at least one photodetector (24); and
- performing optical communication using at least one of the at least one light source (21) and the at least one photodetector (24) of the PPG sensor (20).

15. Computer program comprising program code means for causing a processor (25) to carry out the step of controlling a light source (21) of a PPG sensor (20) to optically transfer data using the same light source (21) used for measurement of a physiological parameter of a subject (100).
